(19) Europäisches Patentamt
European Patent Office
Office européen des brevets

(11) EP 0 692 243 A1

(12) # DEMANDE DE BREVET EUROPEEN

(43) Date de publication:
**17.01.1996 Bulletin 1996/03**

(51) Int Cl.[6]: **A61K 7/00**, A61K 7/48

(21) Numéro de dépôt: **95401682.0**

(22) Date de dépôt: **13.07.1995**

(84) Etats contractants désignés:
**AT BE CH DE DK ES GB GR IE IT LI LU MC NL PT**

(30) Priorité: **13.07.1994 FR 9408750**

(71) Demandeur: **CASTER**
**F-75008 Paris (FR)**

(72) Inventeur: **Rodelet, Jean-François**
**F-92100 Boulogne-Billancourt (FR)**

(74) Mandataire: **Casalonga, Axel**
**D-80469 München (DE)**

(54) **Compositions dépigmentante à base de liposomes encapsulant un extrait concentré végétal riche en arbutine et de glycosphères d'OPC**

(57) La présente invention concerne une composition dépigmentante cosmétique ou dermatologique comportant dans un milieu aqueux physiologiquement acceptable, au moins :

- une suspension (A) aqueuse de liposomes encapsulant un extrait concentré de plante riche en arbutine; et
- une suspension (B) aqueuse de glycosphères encapsulant un oligomère procyanidolique.

L'invention concerne également un procédé de traitement cosmétique des taches solaires de la peau consistant à appliquer sur celle-ci cette composition, ainsi que son utilisation pour la préparation de formulations dermatologiques pour traiter les taches pigmentaires de la peau et réguler la mélanogénèse.

EP 0 692 243 A1

## Description

La présente invention concerne une composition destinée à la dépigmentation de la peau, à base de liposomes encapsulant un extrait concentré végétal riche en arbutine et de glycosphères d'OPC.

La coloration brune de la peau est donnée par l'accumulation dans l'épiderme d'un pigment macromoléculaire : la mélanine. Ce pigment est synthétisé à partir d'un acide aminé : la tyrosine, par les mélanocytes, cellules spécialisées contenues dans le derme. Le processus de production de mélanine est complexe et fait intervenir une suite de mécanismes et de réactions enzymatiques. Il se produit une série de réactions d'oxydation dans lesquelles les enzymes impliquées sont appelées tyrosinases. On peut schématiquement représenter la transformation de la tyrosine en mélanine de la façon suivante :

TYROSINE

TYROSINASE + $O_2$

DOPA : DIHYDROXYPHENYLALANINE

TYROSINASE + $O_2$

DOPAQUINONE

LEUCODOPACHROME

DOPACHROME

DIHYDROXY INDOLE

INDOL QUINONE

MELANINE

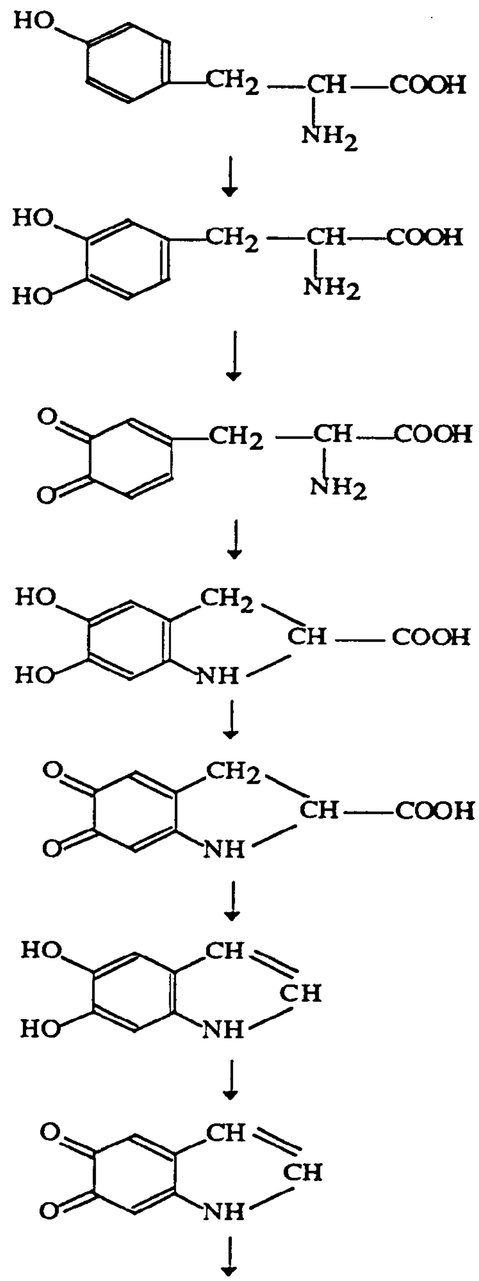

MELANINE

La mélanine constitue une défense naturelle de l'organisme contre les agressions radicalaires.

Les radiations solaires, en particulier les ultraviolets sont porteurs d'énergie et génèrent des radicaux libres dans la peau.

Ces radicaux font partie d'un ecosystème et leur concentration est normalement régulée dans l'organisme par l'action des différentes enzymes : la superoxyde-dismutase, la catalase et la peroxydase. En cas de surproduction de radicaux libres, notamment lors d'exposition au soleil ou d'abus de tabac, le système de régulation enzymatique n'est plus suffisant et les radicaux libres qui ne sont plus neutralisés occasionnent des phénomènes de vieillissement précoce et de nombreuses lésions tissulaires pouvant provoquer l'apparition de maladies graves telles que des troubles cardiovasculaires ou certains cancers.

Toutefois, l'organisme va développer de nouvelles défenses contre ces agressions radicalaires et parmi celles-ci, la production de la mélanine par les mélanocytes, stimulée par la présence de radicaux libres. Un écran absorbant les rayons UV se crée et inhibe la production des radicaux libres.

Mais la production de la mélanine étant d'une grande complexité, on constate fréquemment des anomalies, soit dans la répartition de la mélanine, soit dans l'activité des mélanocytes qui se traduisent visuellement par l'apparition de taches pigmentées souvent disgracieuses pouvant parfois dégénérer et provoquer des maladies graves.

Depuis de nombreuses années, la cosmétologie s'est efforcée de faire disparaître ces taches, soit en les masquant à l'aide d'onguents et de produits de maquillage, soit en essayant de freiner la production de la mélanine.

Dans cet objectif, l'hydroquinone a été largement utilisée. Cette substance active, réductrice énergique, inhibe la réaction d'oxydation catalysée par la tyrosinase. Toutefois, l'utilisation de ce produit actif est actuellement remise en question et sévèrement réglementée en raison de son effet cytotoxique.

On a constaté, en effet, que l'hydroquinone détruisait non seulement les mélanocytes, mais également les kéranocytes et les fibroblastes, provoquant ainsi une dégénérescence de l'épiderme.

Pour ces raisons, l'industrie des produits cosmétiques et dermopharmaceutiques a développé depuis quelques années, l'utilisation de substances dérivées de l'hydroquinone d'origine naturelle, en particulier le 4-hydroxyphényl-β-D-glucopyranoside plus connu sous le nom d'arbutine.

Cette substance n'a pas d'effet cytotoxique et présente des propriétés dépigmentantes; elle exerce une action inhibitrice sur la réaction enzymatique tyrosinase-tyrosine dans le processus de synthèse de la mélanine.

Cette substance est présente dans un certain nombre de plantes telles que le Vaccinium Myrtyllus, la Pirola Umbellata, la Rhododendron Ferrigineum, la Calluna Vulgaris, l'Arctostaphylos Uvae-Ursi ou la Bergenia Cordifolia. Ces plantes contiennent des teneurs plus ou moins importantes en arbutine et sont traditionnellement utilisées en médecine comme antiseptique urinaire.

La demanderesse a découvert d'une manière inattendue qu'en utilisant un extrait de plante concentré, riche en arbutine, encapsulé dans des liposomes en association avec des glycosphères encapsulant un oligomère procyanidolique, on renforçait sensiblement l'action dépigmentante de l'arbutine.

Une telle association permet de traiter efficacement les taches pigmentaires, de réguler la mélanogénèse et permettre ainsi de protéger la peau contre les agressions radicalaires.

L'objet de l'invention est donc une composition dépigmentante cosmétique ou dermatologique, contenant des liposomes renfermant un extrait de plante concentré riche en arbutine et des glycosphères renfermant un oligomère procyanidolique (ou "OPC").

Un autre objet de l'invention consiste en un procédé de traitement cosmétique de la peau, utilisant la composition définie ci-dessous.

On appelle "traitement cosmétique" toute application sur la peau ayant pour effet d'embellir celle-ci, d'améliorer son aspect esthétique.

D'autres objets apparaîtront à la lumière de la description.

Les compositions dépigmentantes, conformes à la présente invention, sont essentiellement caractérisées par le fait qu'elles contiennent dans un milieu aqueux physiologiquement acceptable, au moins :

- une suspension (A) aqueuse de liposomes encapsulant un extrait concentré de plante riche en arbutine; et
- une suspension (B) aqueuse de glycosphères encapsulant un oligomère procyanidolique.

Les suspensions de lyposomes et de glycosphères conformes à l'invention, contiennent préférentiellement le produit actif dans une concentration de 0,2 à 0,8% en poids par rapport au poids de la suspension.

Elle contiennent de préférence entre 5 et 15% en poids de vecteurs liposomes ou glycosphères.

Chaque suspension est incorporée dans les compositions de l'invention dans des proportions comprises de préférence entre 2 et 40% en poids, et plus particulièrement entre 3 et 10% en poids par rapport au poids total de la composition.

On utilise plus particulièrement comme produit actif renfermant de l'arbutine, encapsulé dans les liposomes, un extrait concentré de Bergenia Cordifolia contenant une teneur en arbutine de 25 à 36% en poids.

L'extrait concentré de Bergenia Cordifolia est de préférence obtenu selon le procédé de préparation suivant :

On incorpore une masse M déterminée de feuilles de Bergenia Cordifolia dans un volume d'eau égal à 4 ou 5 fois la masse M, l'eau étant préalablement portée à ébullition. On laisse en contact pendant 1 heure au reflux en maintenant

la température à 95°C ± 5°C. On recueille ensuite le liquide d'extraction par filtration; on presse le résidu; on filtre et on récupère le filtrat Fℓ. On procède à une nouvelle extraction dans les mêmes conditions indiquées ci-dessus, en introduisant une masse M de feuilles Bergenia Cordifolia dans le filtrat Fℓ, précédemment porté à ébullition. On recueille le filtrat final. On concentre ce dernier sous vide jusqu'à obtention d'un extrait mou.

La suspension de liposomes conforme à la présente invention est obtenue par exemple selon le procédé par extrusion à haute pression à travers la presse de French.

Ce procédé est décrit dans l'article de BARENHOLZ Y., AMSELEM S., LICHTENBERG D., A new method for preparation of phospholipid vesicles French press FEBS Lett., 99, 1979, 210-214.

Les produits actifs encapsulés dans les glycosphères sont des oligomères procyanidoliques appelés OPC. Ils sont également appelés pycnogenols. Il s'agit des catéchines et de leurs dérivés possédant la structure flavane-3-ol comme le mentionne l'article de J. MASQUELIER du Journal International de Vitaminologie et de nutrition, 49, n°3, 307-311 (1979).

Ces substances sont extraites de plantes, en particulier d'écorces de conifères telles que le pin, le pin maritime et d'autres espèces de plantes arborescentes ou herbacées. On peut citer à titre d'exemple les proanthocyanidines telles que décrites dans le brevet US-4.698.360.

Les matières premières végétales sont choisies par exemple parmi les pépins de raisins; les sommités fleuries d'aubépine; les bractées de tilleul; l'écorce de pin des Landes; les feuilles de noisetier; les feuilles de ginko biloba; l'écorce de quinquina; la racine de rhubarbe.

Les extraits d'écorce de pin sont particulièrement préférés.

Les oligomères procyanidoliques appelés OPC, encapsulés dans les glycosphères selon la présente invention sont, de préférence, des extraits sous forme de poudre entièrement hydrosolubles obtenus à partir de diverses matières premières végétales selon un procédé comprenant les étapes suivantes :

a) l'épuisement de la matière première végétale par l'eau;

b) la filtration du mélange;

c) la centrifugation du filtrat après séjour au froid;

d) l'épuisement du surnageant par l'acétate d'éthyle, suivi de la séparation de la phase organique contenant l'extrait;

e) la distillation de cette phase sous pression réduite pour récupérer l'acétate d'éthyle;

f) la reprise par l'eau du résidu aqueux;

g) la lyophilisation de la solution aqueuse.

Ce procédé est décrit dans la demande de brevet français n° 8901947.

Les glycosphères encapsulant les composés OPC sont des nanosphères ayant un diamètre moyen de 200 nm, possèdant un noyau central constitué de polysaccharides maillés d'origine végétale, autour duquel peuvent s'organiser des lipides naturels, tels qu'acides gras, phospholipides ou céramides.

Afin de stabiliser à l'intérieur de la particule des actifs hydrophiles, le noyau central hydrophile est dérivé de façon covalente par des charges ioniques permettant d'établir une liaison colombienne entre l'actif et la particule.

Les glycosphères simulent parfaitement la structure des corneocytes, cellules spécialisées situées dans les premières couches de l'épiderme, autour desquelles sont organisés des lipides, principalement les céramides.

Ces vecteurs assurent d'une part la stabilité des oligomères procyanidoliques qui ont tendance à se dégrader au contact de certains constituants des formulations cosmétiques, en conduisant les sous-produits fortement colorés. D'autre part, ils s'intègrent parfaitement parmi les cellules du stratum corneum de l'épiderme, de manière contrôlée, répartissant les produits actifs de façon homogène. Les glycosphères se présentent sous forme de suspension aqueuse.

On utilise plus particulièrement les suspensions de glycosphères encapsulant des composés OPC vendues par la Société A & S BIOVECTEURS.

Les compositions conformes à l'invention se présentent de préférence sous forme de crème, de gel ou de lait.

Le milieu physiologiquement acceptable des compositions est constitué principalement d'eau. Des additifs habituellement utilisés en cosmétique ou dermopharmacie peuvent y être incorporés tels que des agents épaississants, des parfums, des conservateurs, des émulsifiants, des huiles végétales ou minérales, des agents antiseptiques, des agents acidifiants ou alcalinisants.

Le pH des compositions de l'invention est de préférence compris entre 6 et 7.

Un autre objet de l'invention est un procédé de traitement cosmétique de la peau pour traiter les taches solaires,

qui consiste à appliquer sur celles-ci une composition telle que définie ci-dessus à une dose efficace.

Un autre objet de l'invention est l'utilisation de la composition de l'invention pour la préparation d'une formulation dermatologique pour traiter les taches pigmentaires de' la peau et réguler la production de la mélanine.

Les compositions cosmétiques ou dermatologiques sont de préférence appliquées deux fois par jour à des doses comprises de préférence entre 1 et 2,5 mg par $cm^2$ de peau.

Les exemples qui suivent servent à illustrer la présente invention sans toutefois présenter un caractère limitatif.

**EXEMPLES**

Dans les exemples 1 à 4 définis ci-dessous, la suspension de liposomes de Bergenia Cordifolia est une suspension aqueuse à 10% de liposomes du type phospholipides multilamellaires de taille moyenne comprise entre 25 et 50 mm, préparée selon un procédé classique, tel que le procédé par extrusion à haute pression à travers la presse de French.

Ces liposomes contiennent un extrait aqueux de Bergenia Cordifolia dont la teneur en arbutine est de 25 à 36% en poids.

EXEMPLE 1

**GEL DEPIGMENTANT**

- Suspension de liposomes de Bergenia Cordifolia 30-40 g
- Suspension de glycosphères OPC vendue par la Société A & S BIOVECTEURS 30-40 g
- Polymère carboxyvinylique 0,8-1,0 g
- Triéthanolamine qs pH=6,5
- Antiseptique qs
- Parfum qs
- Eau déminéralisée qsp 100 g

EXEMPLE 2

**CREME DEPIGMENTANTE**

- Stéarate de glycérol autoémulsionnable 8-10 g
- Huile de vaseline fluide 6-8 g
- Huile végétale 5-7 g
- Alcool cétylique 0,5-1,5 g
- Lécithine de soja 1-2 g
- Antiseptique qs
- Parfum qs
- Eau déminéralisée qsp 100 g
- Suspension de liposomes de Bergenia Cordifolia 15 g
- Suspension de glycosphères OPC vendue par la Société A & S BIOVECTEURS 15 g

EXEMPLE 3

**CREME DEPIGMENTANTE**

- Ester de sucrose 6-8 g
- Isostéarate d'isostéaryle 8-10 g
- Myristate d'octyldodécyle 5-8 g
- Alcool cétostéarylique 1-2 g
- Antiseptique qs
- Parfum qs
- Eau déminéralisée qsp 100 g
- Suspension de liposomes de Bergenia Cordifolia 3 g
- Suspension de glycosphères OPC vendue par la Société A & S BIOVECTEURS 3 g

EXEMPLE 4

**LAIT DEPIGMENTANT**

- Stéarate de PEG-6 4-6 g
- Alcool cétylique éthoxylé 0,5-1 g
- Huile de vaseline fluide 6-10 g
- Triglycérides d'acides caprylique/caprique 4-6 g
- Antiseptique qs
- Parfum qs
- Gomme de xanthane 0,1-0,5 g
- Eau déminéralisée qsp 100 g
- Suspension de liposomes de Bergenia Cordifolia 5 g
- Suspension de glycosphères OPC vendue par la Société A & S BIOVECTEURS 5 g

ETUDE OUANTITATIVE DE L'EFFET DEPIGMENTANT D'UNE COMPOSITION ANTI-TACHES APPLIQUEE IN VIVO SUR LA PEAU.

On met en évidence, in vivo sur la peau humaine, les variations de la couleur des taches solaires sous l'effet de la crème dépigmentante de l'exemple 3.

On applique cette composition à raison de deux applications quotidiennes pendant 6 semaines.

On effectue les essais sur 5 personnes adultes volontaires (âgées de plus de 18 ans). On mesure la couleur de la peau à l'aide d'un chromamètre MINOLTA de type CR 200 muni d'une cellule de 8 mm.

Le chromamètre convertit les couleurs situées dans la plage de perception humaine dans un code numérique composé de trois paramètres.

L représente la clarté (du sombre au pâle)
a représente la gamme des verts aux rouges
b représente la gamme des bleus aux jaune
C représente la saturation; il est calculé selon la formule

$$C = \sqrt{a^2+b^2}$$

a et b sont des paramètres de chrominance
et L et C sont des paramètres de luminance.

Il devient alors possible d'exprimer dans les moindres détails les différences entre deux zones cutanées qui paraissent être de la même couleur. Après calibration, les mesures sont réalisées directement sur la peau à l'aide d'une source de lumière Xenon pulsée et d'un système à double faisceaux pour mesurer la lumière émise et corriger toute légère déviation.

On détermine les variations en pourcentage de chaque paramètre L, a, b et C des couleurs des taches présentes sur chaque volontaire.

La moyenne des mesures est effectuée sur un ensemble de trois taches déterminées sur une zone traitée et une zone non traitée de chaque volontaire. Les variations en pourcentage sont calculées à partir de la moyenne des trois taches.

On mesure, pour chaque volontaire et pour chaque temps mesuré, les valeurs du paramètre L, a, b ou C avant et après traitement sur la zone traitée et la zone non traitée symétrique.

Les variations en pourcentage d'un paramètre P sont calculées selon la formule :

$$\frac{(P_{zt} - P_{zto}) - (P_{znt} - P_{znto})}{P_{znto} \times 100}$$

avec

$P_{zt}$ : paramètre mesuré après 6 semaines de traitement sur la zone traitée

$P_{zto}$ : paramètre mesuré avant traitement sur la zone traitée

$P_{znto}$ : paramètre mesuré avant traitement sur la zone non traitée

$P_{znt}$ : paramètre mesuré après traitement sur la zone non traitée

Les résultats sont indiqués dans le tableau suivant :

| Paramètres | Moyenne sur 5 volontaires de la variation en pourcentage du paramètre après 6 semaines de traitement |
|---|---|
| L | + 3,33 |
| a | 0 |
| b | - 8,62 |
| C | - 8,04 |

On constate, après six semaines de traitement, que les couleurs des taches solaires sont sensiblement

- plus claires
- moins jaunes
- moins intenses

**Revendications**

1. Composition cosmétique ou dermatologique destinée à la dépigmentation de la peau, caractérisée par le fait qu'elle

comporte dans un milieu aqueux physiologiquement acceptable, au moins :

- une suspension (A) aqueuse de liposomes encapsulant un extrait concentré de plante riche en arbutine; et
- une suspension (B) aqueuse de glycosphères encapsulant un oligomère procyanidolique.

2. Composition selon la revendication 1, caractérisée par le fait que chacune des suspensions (A) et (B) contient le produit actif dans une concentration comprise entre 0,2 et 0,8% en poids par rapport au poids de la suspension.

3. Composition selon la revendication 1 ou 2, caractérisée par le fait que chacune des suspensions (A) et (B) contient de 5 à 15% en poids de liposomes ou de glycosphères.

4. Composition selon l'une quelconque des revendications 1 à 3, caractérisée par le fait qu'elle contient chacune des suspensions (A) et (B) dans une concentration comprise entre 2 et 40% en poids par rapport au poids total de la composition.

5. Composition selon l'une quelconque des revendications 1 à 4, caractérisée par le fait que la suspension (A) est une suspension de liposomes encapsulant un extrait concentré de Bergenia Cordifolia présentant une teneur en arbutine de 25 à 36%.

6. Composition selon l'une quelconque des revendications 1 à 5, caractérisée par le fait qu'elle se présente sous forme de crème, de gel ou de lait.

7. Composition selon l'une quelconque des revendications 1 à 6, caractérisée par le fait que le milieu aqueux physiologiquement acceptable est constitué d'eau et comprend éventuellement des additifs cosmétiques ou dermatologiques choisis parmi les agents épaississants, les parfums, les conservateurs, les émulsifiants, les huiles végétales ou minérales, les agents antiseptiques, des agents acidifiants ou alcalinisants.

8. Procédé de traitement cosmétique des taches solaires sur la peau, caractérisé par le fait que l'on applique sur la peau une composition selon l'une quelconque des revendications 1 à 7.

9. Utilisation de la composition telle que définie selon l'une quelconque des revendications 1 à 7, pour la préparation d'une formulation dermatologique pour traiter les taches pigmentaires de la peau et/ou réguler la mélanogénèse.

Office européen des brevets

# RAPPORT DE RECHERCHE EUROPEENNE

Numero de la demande
EP 95 40 1682

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | PATENT ABSTRACTS OF JAPAN<br>vol. 14, no. 370 (C-747) (4313)<br>& JP-A-02 134 309 (KIKKOMAN CORP)<br>* abrégé *<br>--- | 1-9 | A61K7/00<br>A61K7/48 |
| A | WO-A-93 24106 (L'OREAL)<br>* le document en entier *<br>--- | 1-9 | |
| A | WO-A-93 15708 (L'OREAL)<br>* tableau 1, page 13 , paragraphe: depigmentant; revendications 1-18 *<br>--- | 1-9 | |
| A | S.T.N., Serveur de Bases de Données, Karlsruhe,<br>DE, Fichier Chemical Abstracts, vol106, n 89992<br>* résumé *<br>--- | 1-9 | |
| A | HOUSEHOLD AND PERSONAL PRODUCTS IND.,<br>vol.31, no.5, Mai 1994, US<br>pages 84- - 88<br>DELRIEU ET AL. 'free-radical scavenging activity of procyanidolic oligomers in glycospheres'<br>* en entier *<br>----- | 1-9 | DOMAINES TECHNIQUES RECHERCHES (Int.Cl.6)<br>A61K |

Le présent rapport a été établi pour toutes les revendications

| Lieu de la recherche | Date d'achèvement de la recherche | Examinateur |
|---|---|---|
| LA HAYE | 30 Octobre 1995 | Fischer, J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)